(19) Europäisches Patentamt
European Patent Office
Office européen des brevets

(11) **EP 4 331 633 B1**

(12) **EUROPEAN PATENT SPECIFICATION**

(45) Date of publication and mention of the grant of the patent:
**24.06.2026 Bulletin 2026/26**

(21) Application number: **23746976.2**

(22) Date of filing: **25.01.2023**

(51) International Patent Classification (IPC):
***A61L 27/56*** *(2006.01)* ***A61L 27/02*** *(2006.01)*
***A61L 27/18*** *(2006.01)* ***A61L 27/20*** *(2006.01)*
***A61L 27/22*** *(2006.01)* ***A61L 27/52*** *(2006.01)*
***C08G 65/32*** *(2006.01)* ***C08G 81/00*** *(2006.01)*
***C08J 3/03*** *(2006.01)* ***C08L 71/02*** *(2006.01)*
***C08J 3/24*** *(2006.01)* ***C08J 3/075*** *(2006.01)*

(52) Cooperative Patent Classification (CPC):
(C-Sets available)
**C08L 71/02; A61L 27/18; A61L 27/52; A61L 27/56; C08J 3/03; C08J 3/075; C08J 3/246;** C08J 2371/02; C08J 2405/08; C08J 2471/02; C08J 2489/00
(Cont.)

(86) International application number:
**PCT/JP2023/002236**

(87) International publication number:
**WO 2023/145765 (03.08.2023 Gazette 2023/31)**

(54) **METHOD FOR PRODUCING HYDROGEL HAVING POROUS STRUCTURE**

VERFAHREN ZUR HERSTELLUNG EINES HYDROGELS MIT PORÖSER STRUKTUR

PROCÉDÉ DE PRODUCTION D’HYDROGEL À STRUCTURE POREUSE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **31.01.2022 JP 2022013301**

(43) Date of publication of application:
**06.03.2024 Bulletin 2024/10**

(73) Proprietors:
- **The University of Tokyo**
  **Bunkyo-ku, Tokyo 113-8654 (JP)**
- **Gellycle Co., Ltd.**
  **Tokyo 113-0033 (JP)**

(72) Inventors:
- **SAKAI, Takamasa**
  **Tokyo 113-8654 (JP)**
- **ISHIKAWA, Shohei**
  **Tokyo 113-8654 (JP)**
- **KAMATA, Hiroyuki**
  **Tokyo 113-0033 (JP)**
- **MASUI, Kosuke**
  **Tokyo 113-0033 (JP)**

(74) Representative: **Symbiosis IP Limited**
**The Innovation Centre**
**217 Portobello**
**Sheffield, South Yorkshire S1 4DP (GB)**

(56) References cited:
**WO-A1-2010/070775** **WO-A1-2012/018718**
**WO-A1-2020/027016** **WO-A1-2021/225144**
**WO-A1-2022/019182** **US-A1- 2006 105 420**
**US-A1- 2023 218 797**

- **KATASHIMA TAKUYA ET AL: "Mechanical properties of doubly crosslinked gels", POLYMER JOURNAL, NATURE PUBLISHING GROUP UK, LONDON, vol. 51, no. 9, 22 May 2019 (2019-05-22), pages 851 - 859, XP036861365, ISSN: 0032-3896, [retrieved on 20190522], DOI: 10.1038/S41428-019-0203-6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 331 633 B1

- **BRANDL F ET AL: "Poly(Ethylene Glycol) Based Hydrogels for Intraocular Applications", ADVANCE ENGINEERING MATERIALS, WILEY VCH VERLAG, WEINHEIM, DE, vol. 9, no. 12, 19 December 2007 (2007-12-19), pages 1141 - 1149, XP072139256, ISSN: 1438-1656, DOI: 10.1002/ ADEM.200700221**
- **ISHIKAWA SHOHEI ET AL: "Injectable hydrogels with phase-separated structures that can encapsulate live cells", BIORXIV, 1 February 2022 (2022-02-01), pages 1 - 28, XP093262780, Retrieved from the Internet <URL:https://doi.org/ 10.1101/2022.01.31.478579;> [retrieved on 20250324], DOI: 10.1101/2022.01.31.478579**
- **ISHIKAWA ASUMI, IKEDA NAMIE, MAEDA SHUICHI, FUJII KENTA: "Polymer network formation mechanism of multifunctional poly(ethylene glycol)s in ionic liquid electrolyte with a lithium salt", PHYSICAL CHEMISTRY CHEMICAL PHYSICS, vol. 23, no. 31, 12 August 2021 (2021-08-12), pages 16966 - 16972, XP093080608, ISSN: 1463-9076, DOI: 10.1039/ D1CP02710G**

(52) Cooperative Patent Classification (CPC): (Cont.)

C-Sets

**A61L 27/18, C08L 71/02;**
**C08L 71/02, C08L 71/02**

## Description

### Technical Field

The present invention relates to a method for producing a hydrogel having a porous structure.

### Background Art

In recent years, hydrogels having a polymer network structure have characteristics such as excellent water retention ability and biocompatibility, and thus the hydrogels are materials that are expected to be applied to various use applications such as medical use applications including artificial tissues, scaffold materials for regeneration, sealants, adhesion preventing materials, drug delivery substrates, contact lenses, and the like, and use applications for sensors and surface coating materials (for example, Non Patent Literature 1). In particular, in use applications such as scaffold materials for regeneration, it is desired to develop a polymer material having a μm-scale porous structure.

However, conventionally, in order to obtain the μm-scale porous structure, it has been necessary that a polymer material is produced using a top-down method such as microfabrication of a hydrogel or polymer structure produced in advance by lithography or the like, or a solvent-insoluble polymer raw material. On the other hand, when a solvophilic polymer raw material is used, the material itself is dissolved in the solvent in the first place, or only a hydrogel having a small (nm-scale) porous structure can be produced.

### Citation List

### Non Patent Literature

Non Patent Literature 1: Sakai et al., Macromolecules, 41, 5379-5384, 2008
Non Patent Literature 2: Katashima et al., Polymer Journal, 51(9), 851-859, 2019

### Summary of Invention

### Technical Problem

Therefore, an object of the present invention is to provide a hydrogel material having a μm-scale porous structure, and to provide a production method suitable for producing the hydrogel material from a solvophilic polymer raw material.

### Solution to Problem

As a result of diligent studies to solve the above problems, the present inventors have found that a hydrogel having a μm-scale porous structure can be formed by a simple method by performing a gelation reaction in the presence of a high-concentration alkali metal salt, and have completed the present invention. Such a hydrogel has a sponge-like three-dimensional network structure (porous structure) formed by spontaneous phase separation of a polymer as a constituent component in a production process.

The present invention in its various aspects is as set out in the accompanying claims.

According to a first aspect, the present invention provides as follows:

A method for producing a hydrogel having a porous structure, the production method including steps of:

a) preparing a first solution in which a compound A is dissolved in an aqueous solvent containing an alkali metal salt at a concentration of 0 to 1000 mM;
b) preparing a second solution in which a compound B chemically crosslinkable with the compound A is dissolved in an aqueous solvent containing an alkali metal salt at a concentration of 0 to 1000 mM;
c) mixing the first solution and the second solution to obtain a gelling solution containing one or more alkali metal salts at a concentration of 300 mM to 1000 mM; and
d) forming a chemical crosslinking by the compound A and the compound B in the gelling solution regardless of the presence or absence of an external stimulus to obtain a hydrogel having a porous structure,

wherein at least one of the compounds A and B is a linear, tri-branched, tetra-branched, or octa-branched polyethylene glycol (PEG) having a total of two or more nucleophilic functional groups or electrophilic functional groups in a side chain or at a terminal;
wherein the weight average molecular weight of the PEG is in the range of $2\times10^3$ to $100\times10^3$ Da, and
wherein a time from preparation of the gelling solution to solidification of the gelling solution is 1 second or longer.

In preferred embodiments, the alkali metal salt is selected from the group consisting of sodium dihydrogen phosphate, disodium hydrogen phosphate, sodium citrate, sodium acetate, potassium sulfate, sodium sulfate, and any combination thereof;

In preferred embodiments, the hydrogel has a maximum value of transmittance at a wavelength of 405 nm of 80% or less;
In preferred embodiments, the hydrogel contains the polyethylene glycol in a range of 20 g/L to 250 g/L;
Preferably, when in a mixed solution of the first solution and the second solution, an equilibrium swelling degree of a hydrogel formed under a condition of a concentration of the alkali metal salt of 200 mM is designated as $Q_{low}$, and an equilibrium swelling degree of a hydrogel formed in the presence of an arbitrary concentration within 300 mM to 1000 mM of the alkali metal salt is designated as Q, Q satisfies the following relational expression:

$$Q \geq 1.1*Q_{low};$$

wherein the equilibrium swelling degree is defined as $(d_eq/d_0)^3$,
wherein "d_eq" is the diameter of the hydrogel in an equilibrium state, and "d_0" is the diameter of the hydrogel immediately after preparation.

Preferably, when in a mixed solution of the first solution and the second solution, a tensile stress at break of a hydrogel formed under a condition of a concentration of the alkali metal salt of 200 mM is designated as $\sigma_{low}$, and a tensile stress at break of a hydrogel formed in the presence of an arbitrary concentration within 300 mM to 1000 mM of the alkali metal salt is designated as $\sigma$, $\sigma$ satisfies the following relational expression:

$$\sigma \geq 1.5*\sigma_{low};$$

wherein the tensile stress at break is measured by means of a tensile test.
Preferably, one of the compounds A and B is PEG and the other is composed of one or more selected from the group consisting of PEG, a peptide, a protein, chitosan, hyaluronic acid, and derivatives thereof; and
In preferred embodiments, the external stimulus in the step d) is light irradiation.
In another aspect, the present invention also relates to
A kit for producing a hydrogel having a porous structure, the kit containing:

a first solution in which a compound A is dissolved in an aqueous solvent containing an alkali metal salt at a concentration of 0 to 1000 mM; and
a second solution in which a compound B chemically crosslinkable with the compound A is dissolved in an aqueous solvent containing an alkali metal salt at a concentration of 0 to 1000 mM,
wherein a concentration of the alkali metal salt when the first solution and the second solution are mixed is in a range of 300 mM to 1000 mM in total, and
at least one of the compounds A and B is a linear, tri-branched, tetra-branched, or octa-branched polyethylene glycol (PEG) having a total of two or more nucleophilic functional groups or electrophilic functional groups in a side chain or at a terminal.
wherein the weight average molecular weight of the PEG is in the range of $2\times10^3$ to $100\times10^3$ Da, and
wherein a time from preparation of the gelling solution to solidification of the gelling solution is 1 second or longer

Advantageous Effects of Invention

According to the production method of the present invention, by adjusting an alkali metal salt concentration in a polymer solution, a hydrogel having a μm-scale porous structure can be easily and efficiently obtained only by mixing the solution.
Since the hydrogel obtained by the production method of the present invention forms a sponge-like, μm-scale three-dimensional network structure (porous structure), the hydrogel may be a material suitable for cell infiltration and

adhesion.

## Brief Description of Drawings

Fig. 1 is an image showing a state of gelation under conditions of an alkali metal salt concentration of 200 mM and a PEG concentration of 100 g/L.
Fig. 2 is an image showing a state of gelation under conditions of an alkali metal salt concentration of 400 mM and a PEG concentration of 100 g/L.
Fig. 3 is an image of hydrogels (PEG concentration: 20, 50, 80, and 100 g/L) each prepared at a different total alkali metal salt concentration.
Fig. 4 is a graph showing transmittance at 405 nm in hydrogels (PEG concentration: 20, 50, 80, and 100 g/L) each prepared at a different total alkali metal salt concentration.
Fig. 5 is an image showing a state of a hydrogel immediately after preparation (upper part, scale bar: 10 mm) and a confocal microscope image of the hydrogel (lower part, scale bar: 20 μm).
Fig. 6 is an image showing a state of a hydrogel in an equilibrium state in water (upper part, scale bar: 10 mm) and a confocal microscope image of the hydrogel (lower part, scale bar: 20 μm).
Fig. 7 is a graph showing an equilibrium swelling degree of a hydrogel prepared at each total alkali metal salt concentration.
Fig. 8 is a graph showing a tensile stress at break of a hydrogel prepared at each total alkali metal salt concentration.

## Description of Embodiments

Hereinafter, embodiments of the present invention will be described.

### (1) Method for Producing Hydrogel

A production method of the present invention is a method for producing a hydrogel having a porous structure, the production method including the following steps of a) to d):

a) preparing a first solution in which a compound A is dissolved in an aqueous solvent containing an alkali metal salt at a concentration of 0 to 1000 mM;
b) preparing a second solution in which a compound B chemically crosslinkable with the compound A is dissolved in an aqueous solvent containing an alkali metal salt at a concentration of 0 to 1000 mM;
c) mixing the first solution and the second solution to obtain a gelling solution containing one or more alkali metal salts at a concentration of 300 mM to 1000 mM; and
d) forming a chemical crosslinking by the compound A and the compound B in the gelling solution regardless of the presence or absence of an external stimulus to obtain a hydrogel having a porous structure.

One of the compounds A and B is a linear (that is, bi-branched), tri-branched, tetra-branched, or octa-branched polyethylene glycol (PEG) having a total of two or more nucleophilic functional groups or electrophilic functional groups in a side chain or at a terminal.

The hydrogel obtained by the production method of the present invention has a sponge-like three-dimensional network structure (porous structure) formed by spontaneous phase separation of a polymer as a constituent component in a production process (hereinafter, such a structure may be referred to as "sponge-like porous structure"). It is also characterized in that the mesh size is on the order of μm much larger than the order of nm obtained in conventional hydrogels. In this hydrogel, a polymer unit is not observed in voids forming a porous structure. That is, the hydrogel as a whole does not have two or more regions where the polymer density is greatly different. Since the polymer unit is not present in the voids, when the hydrogel is compressed by an external force, water can be discharged like a so-called normal sponge. In the production process in the present invention, some or all of the polymer units to be used are not spontaneously solvophilic after a certain period of time. From that time point, the non-solvophilic polymer unit is crosslinked, and as a result, a phase separation structure containing a non-solvophilic polymer as a constituent component is obtained.

In the present specification, the "gel" is generally a dispersion system of a polymer that has high viscosity and lost fluidity, and refers to a state having a relationship of G' ≥ G" between a storage modulus G' and a loss modulus G". In particular, when a solvent contained in the gel is an aqueous solvent, the gel is referred to as a "hydrogel".

Hereinafter, the compounds A and B constituting the hydrogel of the present invention and various conditions in

various steps will be described.

1-a. Compounds A and B (Gel Constituent Components)

The compounds A and B which are components for forming the hydrogel of the present invention are typically solvophilic polymer species, that is, when the gel is a hydrogel, as the compounds A and B, hydrophilic polymer species can be used. As for the polymer species, polymer species known in the technical field can be used in accordance with the use application, and shape of a final hydrogel as long as they are capable of forming a hydrogel by a gelation reaction (such as a crosslinking reaction) in a solution regardless of the presence or absence of an external stimulus. More specifically, polymer units capable of forming a network structure, particularly a three-dimensional network structure, by crosslinking of the polymer species with each other in the final hydrogel are preferable.

The hydrophilic polymers used as the compounds A and B can be preferably polymers having a polyethylene glycol backbone or a polyvinyl backbone. Typical examples of the polymers having a polyethylene glycol backbone include polymers having a plurality of branches of polyethylene glycol backbones such as two branches, three branches, four branches, or eight branches, and in particular, polymer species having four branches of polyethylene glycol backbones are preferable. Such a gel including a tetra-branched polyethylene glycol backbone is known as a Tetra-PEG gel, and a network-structure network is constructed by an AB-type cross-end coupling reaction between two types of tetra-branched polymers having an electrophilic functional group such as an active ester structure and a nucleophilic functional group such as an amino group at each terminal (Matsunaga et al., Macromolecules, Vol. 42, No. 4, pp. 1344-1351, 2009). Tetra-PEG gels can also be prepared on site easily by simple two-liquid mixing of each polymer solution. By using the Tetra-PEG technique, the gelation time can also be controlled by adjusting the pH and ionic strength during hydrogel preparation. Since the obtained hydrogel contains PEG as a main component, the hydrogel is also excellent in biocompatibility.

Polymers having other than a polyethylene glycol backbone can also be used as long as they can be gelled by crosslinking with each other, and for example, polymers having a polyvinyl backbone such as methyl methacrylate can also be used.

In the production method of the present invention, one of the compounds A and B is a linear, tri-branched, tetra-branched, or octa-branched polyethylene glycol (PEG) having a total of two or more nucleophilic functional groups or electrophilic functional groups in a side chain or at a terminal.

In a preferred embodiment, one of the compounds A and B is PEG and the other can be composed of one or more selected from the group consisting of PEG, a peptide, a protein, a polysaccharide. Examples of the protein include gelatin and albumin. Examples of the polysaccharide include chitosan, hyaluronic acid, and derivatives thereof.

The molecular weight of molecules other than PEG of the compounds A and B is not particularly limited. On the other hand, the molecular weight of PEG is in the range of $2\times10^3$ to $100\times10^3$ Da, preferably $5\times10^3$ to $40\times10^3$ Da, and more preferably $10\times10^3$ to $20\times10^3$ Da. Since PEG tends to be more easily phase-separated as the molecular weight is larger, when the molecular weight is too large, phase separation may occur in a solution state before the crosslinking reaction, which is not preferable. In the present invention, a hydrogel having a porous structure can be obtained by setting the molecular weights of the compounds A and B within a predetermined range and setting the concentration of the alkali metal salt described below within a predetermined range.

In a typical embodiment, both the compounds A and B can be PEG. In this case, the compound A can be a first polymer unit having a total of two or more nucleophilic functional groups in a side chain or at a terminal, the compound B can be a second polymer unit having a total of two or more electrophilic functional groups in a side chain or at a terminal, and a means for reacting these two types of polymer species to crosslink them is suitable. The total of the nucleophilic functional group and the electrophilic functional group is preferably 5 or more. These functional groups are preferably present at a terminal. The content of the first polymer unit can be also larger than the content of the second polymer unit, or the content of the second polymer unit can be larger than the content of the first polymer unit.

Examples of the nucleophilic functional group present in the polymer unit include a thiol group (-SH) (also referred to as a sulfhydryl group), and an amino group, and those skilled in the art can appropriately use a known nucleophilic functional group. Preferably, the nucleophilic functional group is a thiol group or an amino group. The nucleophilic functional groups may be the same as or different from each other, but are preferably the same. When the functional groups are the same, reactivity with an electrophilic functional group that forms a crosslinking bond becomes uniform, and a hydrogel having a homogeneous three-dimensional structure is easily obtained.

As the electrophilic functional group present in the polymer unit, a maleimidyl group or an active ester group can be used. Examples of the active ester group include an N-hydroxy-succinimidyl (NHS) group and a sulfosuccinimidyl group. Those skilled in the art can appropriately use other known electrophilic functional groups. Preferably, the electrophilic functional group is a maleimidyl group. The electrophilic functional groups may be the same as or different from each other, but are preferably the same. When the functional groups are the same, reactivity with a nucleophilic functional group that forms a crosslinking bond becomes uniform, and a hydrogel having a homogeneous three-dimensional structure is easily obtained.

In addition to the combination of the nucleophilic functional group and the electrophilic functional group, a covalent bond forming reaction known to those skilled in the art, such as a cycloaddition reaction with azide and alkyne, or other reaction called click chemistry, can also be appropriately used.

Preferred non-limiting specific examples of the polymer unit having a nucleophilic functional group at a terminal include a compound represented by the following Formula (I) having four branches of a polyethylene glycol backbone and having a thiol group at a terminal.

[Chemical Formula 1]

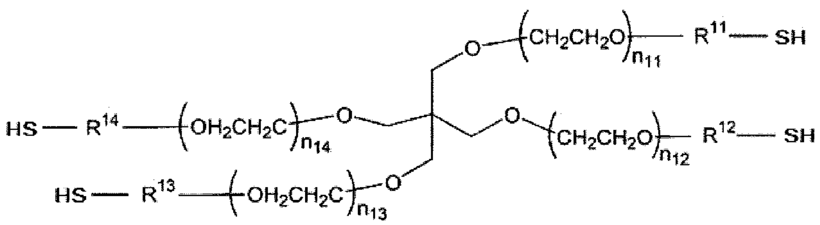

(I)

$n_{11}$ to $n_{14}$ may be the same as or different from each other. As the values of $n_{11}$ to $n_{14}$ are closer to each other, a homogeneous three-dimensional structure can be obtained, and the strength becomes higher. Therefore, in order to obtain a high-strength hydrogel, $n_{11}$ to $n_{14}$ are preferably the same. When the values of $n_{11}$ to $n_{14}$ are too high, the strength of the gel is weak, and when the values of $n_{11}$ to $n_{14}$ are too low, the gel is hardly formed due to steric hindrance of the compound. Therefore, $n_{11}$ to $n_{14}$ are, for example, an integer value of 11 to 569, preferably 28 to 228, and more preferably 56 to 114. The molecular weight thereof is, for example, $2\times10^3$ to $100\times10^3$ Da,
preferably $5\times10^3$ to $40\times10^3$ Da, and more preferably $10\times10^3$ to $20\times10^3$ Da.

In Formula (I), $R^{11}$ to $R^{14}$ are linker moieties connecting a functional group and a core moiety. $R^{11}$ to $R^{14}$ may be the same as or different from each other, but are preferably the same in order to produce a high-strength hydrogel having a homogeneous three-dimensional structure. $R^{11}$ to $R^{14}$ represent a $C_1$-$C_7$ alkylene group, a $C_2$-$C_7$ alkenylene group, -NH-$R^{15}$-, -CO-$R^{15}$-, -$R^{16}$-O-$R^{17}$-, -$R^{16}$-NH-$R^{17}$-, -$R^{16}$-$CO_2$-$R^{17}$-, -$R^{16}$-$CO_2$-NH-$R^{17}$-, -$R^{16}$-CO-$R^{17}$-, $R^{16}$-NH-CO$R^{17}$-, or -$R^{16}$-CO-NH-$R^{17}$-. $R^{15}$ represents a $C_1$-$C_7$ alkylene group. $R^{16}$ represents a $C_1$-$C_3$ alkylene group. $R^{17}$ represents a $C_1$-$C_5$ alkylene group.

The "$C_1$-$C_7$ alkylene group" means an optionally branched alkylene group having 1 or more and 7 or less carbon atoms, and means a linear $C_1$-$C_7$ alkylene group or a $C_2$-$C_7$ alkylene group having one or two or more branches (the number of carbon atoms including the branch is 2 or more and 7 or less). Examples of the $C_1$-$C_7$ alkylene group include a methylene group, an ethylene group, a propylene group, and a butylene group. Examples of the $C_1$-$C_7$ alkylene group include $-CH_2-$, $-(CH_2)_2-$, $-(CH_2)_3-$, $-CH(CH_3)-$, $-(CH_2)_3-$, $-(CH(CH_3))_2-$, $-(CH_2)_2-CH(CH_3)-$, $-(CH_2)_3-CH(CH_3)-$, $-(CH_2)_2-CH(C_2H_5)-$, $-(CH_2)_6-$, $-(CH_2)_2-C(C_2H_5)_2-$, and $-(CH_2)_3C(CH_3)_2CH_2-$.

The "$C_2$-$C_7$ alkenylene group" is a linear or branched alkenylene group having 2 to 7 carbon atoms having one or two or more double bonds in the chain, and examples thereof include divalent groups having double bonds formed by eliminating 2 to 5 hydrogen atoms of adjacent carbon atoms from the alkylene group.

On the other hand, preferred non-limiting specific examples of the polymer unit having an electrophilic functional group at a terminal include a compound represented by the following Formula (II) having four branches of a polyethylene glycol backbone and having a maleimidyl group at a terminal.

[Chemical Formula 2]

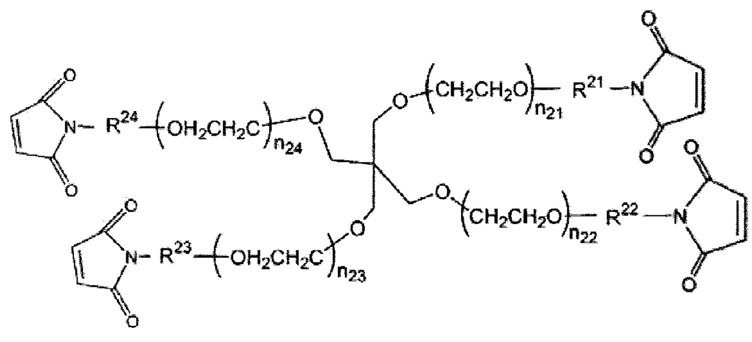

(II)

In Formula (II), $n_{21}$ to $n_{24}$ may be the same as or different from each other. As the values of $n_{21}$ to $n_{24}$ are closer to each other, the hydrogel can have a homogeneous three-dimensional structure and has high strength, which is preferable, and $n_{21}$ to $n_{24}$ are preferably the same. When the values of $n_{21}$ to $n_{24}$ are too high, the strength of the gel is weak, and when the values of $n_{21}$ to $n_{24}$ are too low, the gel is hardly formed due to steric hindrance of the compound. Therefore, $n_{21}$ to $n_{24}$ are, for example, an integer value of 11 to 569, preferably 28 to 228, and more preferably 56 to 114. The molecular weight of a second tetra-branched compound of the present invention is, for example, $2\times10^3$ to $100\times10^3$ Da, preferably $5\times10^3$ to $40\times10^3$ Da, and more preferably $10\times10^3$ to $20\times10^3$ Da.

In Formula (II), $R^{21}$ to $R^{24}$ are linker moieties connecting a functional group and a core moiety. $R^{21}$ to $R^{24}$ may be the same as or different from each other, but are preferably the same in order to produce a high-strength gel having a homogeneous three-dimensional structure. In Formula (II), $R^{21}$ to $R^{24}$ may be the same as or different from each other, and represent a $C_1$-$C_7$ alkylene group, a $C_2$-$C_7$ alkenylene group, -NH-$R^{25}$-, -CO-$R^{25}$-, -$R^{26}$-O-$R^{27}$-, -$R^{26}$-NH-$R^{27}$-, -$R^{26}$-$CO_2$-$R^{27}$-, -$R^{26}$-$CO_2$-NH-$R^{27}$-, -$R^{26}$-CO-$R^{27}$-, -$R^{26}$-NH-CO$R^{27}$-, or -$R^{26}$-CO-NH-$R^{27}$-. $R^{25}$ represents a $C_1$-$C_7$ alkylene group. $R^{26}$ represents a $C_1$-$C_3$ alkylene group. $R^{27}$ represents a $C_1$-$C_5$ alkylene group.

In the present specification, the alkylene group and the alkenylene group may have one or more arbitrary substituents. Examples of the substituent include, but are not limited to, an alkoxy group, a halogen atom (may be any of a fluorine atom, a chlorine atom, a bromine atom, and an iodine atom), an amino group, a mono- or disubstituted amino group, a substituted silyl group, an acyl group, and an aryl group. When the alkyl group has two or more substituents, these substituents may be the same or different. The same applies to alkyl moieties of other substituents including alkyl moieties (for example, alkyloxy groups aralkyl groups).

In the present specification, when a certain functional group are defined as "optionally substituted", the type of substituent, substitution position, and the number of substituents are not particularly limited, and when there are two or more substituents, these substituents may be the same or different. Examples of the substituent include, but are not limited to, an alkyl group, an alkoxy group, a hydroxyl group, a carboxyl group, a halogen atom, a sulfo group, an amino group, an alkoxycarbonyl group, and an oxo group. Substituents may be further present in these substituents.

1-b. Various Conditions in Each Step

The alkali metal salt used in the first and second solutions can be preferably selected from the group consisting of sodium dihydrogen phosphate, disodium hydrogen phosphate, sodium citrate, sodium acetate, potassium sulfate, sodium sulfate, and any combination thereof. At least one of the first and second solutions contains the alkali metal salt.

When the alkali metal salt concentration of the first solution and the second solution are too high, unintended phase separation occurs, which is not preferable. The concentration of the alkali metal salt in the first solution is in a concentration range of 0 to 1000 mM, preferably 0 to 500 mM, and more preferably 0 to 450 mM. Similarly, the concentration of the alkali metal salt in the second solution is in a concentration range of 0 to 1000 mM, preferably 0 to 500 mM, and more preferably 0 to 450 mM. Typically, the lower limit value of the alkali metal salt concentration in either one or both of the first solution and the second solution can be 0.01 mM.

The concentration of the alkali metal salt in the gelling solution (mixed solution before gelation) obtained by mixing the first solution and the second solution is in a concentration range of 300 to 1000 mM, preferably 350 to 500 mM, and more preferably 400 to 450 mM. When the concentration of the alkali metal salt is lower than the range, the crosslinked polymer unit cannot be phase-separated, and a porous structure is not formed. On the other hand, when the concentration of the alkali metal salt is too high, phase separation occurs in a solution state before the polymer unit is crosslinked, which is not preferable.

At the time of mixing in the step c), when the temperature of each solution is too high, unintended phase separation occurs before the start of the crosslinking reaction, and when the temperature thereof is too low, the fluidity of each solution may deteriorate and it may be difficult to handle each solution. Therefore, it is desirable to control the temperature to preferably 10 to 40°C, more preferably 25 to 35°C, and still more preferably 25°C. For example, the first and second solutions can be mixed in a thermostat bath set at the same temperature as each liquid temperature. After the start of the crosslinking reaction between the compounds A and B, the solution temperature may change to some extent. For example, the first and second solutions can also be directly injected into an in vivo environment such as in vivo to form a porous hydrogel in situ.

The term "regardless of the presence or absence of an external stimulus" in the step d) means that gelation proceeds only by mixing the first solution and the second solution without applying an external light stimulus or heat stimulus. Preferably, the external stimulus is a light stimulus.

In the steps c) and d), a time from preparation of the gelling solution to solidification of the gelling solution is 1 second or longer. This makes it possible to ensure a time for mixing the first solution and the second solution. When the time is faster than this, mixing is not successful, and the hydrogel tends to be formed ununiformly, which is not preferable. From another viewpoint, the time until the gelling solution becomes a solid is preferably 5 seconds or longer. In this case, the gelling solution can be poured into a mold as a liquid or directly injected into an in vivo environment after the gelling solution

is filled in a syringe barrel, and a porous hydrogel can be molded in a desired shape at a desired location.

1-b. Physical Properties etc. of Hydrogel

The hydrogel obtained by the production method of the present invention is characterized by having a porous structure on the order of μm as described above. The pore size varies depending on conditions such as the total alkali metal salt concentration, and can be, for example, a size of 1 to 500 μm, and is preferably 1 to 100 μm from the viewpoint of mechanical properties of the hydrogel. The pore and the size thereof can be observed by various methods, and as an example, the pore and the size thereof can be measured by bonding a fluorescent dye to a polymer constituting the hydrogel and then acquiring a confocal microscope image. In the hydrogel of the present invention, a polymer unit is not substantially observed in voids forming a porous structure. That is, the hydrogel as a whole does not have two or more regions where the polymer density is greatly different. Since the polymer unit is substantially absent in the voids, when the hydrogel is compressed by an external force, water can be discharged like a so-called normal sponge.

The polymer content in the hydrogel of the present invention is not particularly limited, but in a preferred embodiment, polyethylene glycol is contained in a range of 20 g/L to 250 g/L.

The hydrogel of the present invention has a transmittance lower than the transmittance of the polymer unit before gelation. This is because in the hydrogel of the present invention, the polymer as a constituent component spontaneously phase-separates in a production process. As a result, the poorly soluble polymer behaves as if in a state of phase separation in the solvent, and is not completely transparent but becomes cloudy. Preferably, in the hydrogel of the present invention, a maximum value of transmittance in a wavelength range of 405 nm is transmittance of 80% or less. In terms of such transmittance, the hydrogel of the present invention exhibits completely different properties from a normal hydrogel which is almost transparent.

Preferably, when in a mixed solution of the first solution and the second solution, an equilibrium swelling degree of a hydrogel formed under a condition of a concentration of the alkali metal salt of 200 mM is designated as $Q_{low}$, and an equilibrium swelling degree of a hydrogel formed in the presence of an arbitrary concentration of the alkali metal salt is designated as Q, Q satisfies the following relational expression:

$$Q \geq 1.1 * Q_{low}.$$

The hydrogel of the present invention is also characterized in terms of tensile stress at break. Specifically, when in a mixed solution of the first solution and the second solution, a tensile stress at break of a hydrogel formed under a condition of a concentration of the alkali metal salt of 200 mM is designated as $\sigma_{low}$, and a tensile stress at break of a hydrogel formed in the presence of an arbitrary concentration of the alkali metal salt is designated as σ, σ satisfies the following relational expression:

$$\sigma \geq 1.5 * \sigma_{low}.$$

The hydrogel of the present invention can be processed into various shapes such as a thin film according to use applications. Any methods known in this technical field can be used for such processing. For example, in the case of a thin film, the thin film can be obtained, for example, by a method of applying the gelling solution onto a flat substrate such as glass in a state of having fluidity prior to complete solidification.

Examples

Hereinafter, the present invention will be described in more detail with reference to Examples; however, the present invention is not limited thereto. In the following Examples, the unit of g/L of the polymer concentration is used, and 1 g/L corresponds to 0.1 wt%.

1. Preparation of Common Materials

As a buffer solution of the polymer solution in each experiment, a phosphate buffer solution (PB) was used. The PB contains sodium dihydrogen phosphate and disodium hydrogen phosphate as alkali metal salts. The PB can be prepared by a generally reported method, but here, the PB was prepared to have a pH of 7.4 according to a report by Kurakazu et al. (Macromolecules 2010, 43, 8, 3935-3940). At this time, the buffering capacity of the buffer solution was adjusted by changing the molar concentration (alkali metal salt concentration) of the solute in a range of 0 to 1000 mM.

2. Preparation of Hydrogel Using Tetra-Branched Polyethylene Glycol (PEG)

As raw material polymers, Tetra-PEG-SH (tetrasulfhydryl-polyethylene glycol; NOF CORPORATION, trade name PTE-100SH) having a -SH group at a terminal and Tetra-PEG-OSu (tetrasuccinimidyl-polyethylene glycol; NOF CORPORATION, trade name PTE-100HS) having a succinimidyl group at a terminal were used. The weight average molecular weight (Mw) of both the raw material polymers was 10000.

Preparation of First Solution

Tetra-PEG-SH was dissolved in the phosphate buffer solution (PB). At this time, the alkali metal salt concentration of the PB was set to any concentration of 0 to 1000 mM. The PEG concentration was set to any concentration of 20 to 250 g/L.

Preparation of Second Solution

Tetra-PEG-OSu was dissolved in the PB. At this time, the alkali metal salt concentration of the PB was set to any concentration of 0 to 1000 mM. The PEG concentration was set to any concentration of 20 to 250 g/L.

<Comparative Example 1>

The first solution (alkali metal salt concentration: 200 mM, PEG concentration: 100 g/L) and the second solution (alkali metal salt concentration: 200 mM, PEG concentration: 100 g/L) were mixed in the same volume ratio in an empty centrifuge tube. This mixed solution was used as a gelling solution containing PEG at a concentration of 100 g/L and an alkali metal salt at a concentration of 200 mM. The gelling solution (1 mL) was put into a 2.5 mL syringe barrel to which a 27G syringe needle (inner diameter: 0.22 $\pm$ 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 10 minutes without any special external stimulus. When the glass vial was tilted, the gelling solution lost fluidity and became a transparent solid (Fig. 1). The obtained transparent hydrogel did not discharge water even when compressed with a finger. This is a previously reported PEG hydrogel preparation process reported in (Sakai et al., Macromolecules 2008, 41, 14, 5379-5384), and it is an expected result that a transparent hydrogel is obtained.

<Comparative Example 2>

The first solution (alkali metal salt concentration: 200 mM, PEG concentration: 250 g/L) and the second solution (alkali metal salt concentration: 200 mM, PEG concentration: 250 g/L) were mixed in the same volume ratio in an empty centrifuge tube. This mixed solution was used as a gelling solution containing PEG at a concentration of 250 g/L and an alkali metal salt at a concentration of 200 mM. The gelling solution (1 mL) was put into a 2.5 mL syringe barrel to which a 27G syringe needle (inner diameter: 0.22 $\pm$ 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a transparent solid. The obtained transparent hydrogel did not discharge water even when compressed with a finger. It was shown that even if the PEG concentration simply increased as compared to Comparative Example 1, a porous structure was not obtained.

<Example 1>

The first solution (alkali metal salt concentration: 400 mM, PEG concentration: 100 g/L) and the second solution (alkali metal salt concentration: 400 mM, PEG concentration: 100 g/L) were mixed in the same volume ratio in an empty centrifuge tube. This mixed solution was used as a gelling solution containing PEG at a concentration of 100 g/L and an alkali metal salt at a concentration of 400 mM. The gelling solution (1 mL) was put into a 2.5 mL syringe barrel to which a 27G syringe needle (inner diameter: 0.22 $\pm$ 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 10 minutes without any special external stimulus. When the glass vial was tilted, the gelling solution lost fluidity and became a white solid (Fig. 2). When the obtained hydrogel was compressed with a finger, it was observed that water was discharged. This directly indicates that the hydrogel has a porous structure found in normal sponges. This result is

attributed to the phase separation of PEG in the hydrogel due to the presence of the alkali metal salt at a higher concentration than the gel preparation process reported so far.

<Example 2>

The first solution (alkali metal salt concentration: 1000 mM, PEG concentration: 100 g/L) and the second solution (alkali metal salt concentration: 0 mM, PEG concentration: 100 g/L) were mixed in the same volume ratio in an empty centrifuge tube. This mixed solution was used as a gelling solution containing PEG at a concentration of 100 g/L and an alkali metal salt at a concentration of 500 mM. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged.

Similarly, the first solution (alkali metal salt concentration: 0 mM, PEG concentration: 100 g/L) and the second solution (alkali metal salt concentration: 1000 mM, PEG concentration: 100 g/L) were mixed in the same volume ratio in an empty centrifuge tube. This mixed solution was used as a gelling solution containing PEG at a concentration of 100 g/L and an alkali metal salt at a concentration of 500 mM. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged.

These results suggest that it is not essential for the alkali metal salt to be contained in both the first solution and the second solution, and it is sufficient that the alkali metal salt is present in a final gelling solution (mixture).

<Example 3>

The first solution (alkali metal salt concentration: 1000 mM, PEG concentration: 100 g/L) and the second solution (alkali metal salt concentration: 1000 mM, PEG concentration: 100 g/L) were mixed in the same volume ratio in an empty centrifuge tube. This mixed solution was used as a gelling solution containing PEG at a concentration of 100 g/L and an alkali metal salt at a concentration of 1000 mM. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged. As described above, it was confirmed that a porous structure was formed even at a relatively high alkali metal salt concentration.

<Example 4>

The first solution (alkali metal salt concentration: 600 mM, PEG concentration: 20 g/L) and the second solution (alkali metal salt concentration: 600 mM, PEG concentration: 20 g/L) were mixed in the same volume ratio in an empty centrifuge tube. This mixed solution was used as a gelling solution containing PEG at a concentration of 20 g/L and an alkali metal salt at a concentration of 600 mM. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged. It was confirmed that porositization was possible even at a relatively low PEG concentration (for example, 20 g/L).

<Example 5>

In Example 5, sodium dihydrogen phosphate contained in a phosphate buffer solution (PB) and potassium

sulfate ($K_2SO_4$), which is an alkali metal salt different from disodium hydrogen phosphate, were additionally added to PB in advance. Specifically, $K_2SO_4$ was added as a solid to PB prepared so as to have an alkali metal salt concentration of 10 mM so that the $K_2SO_4$ concentration was 500 mM, and $K_2SO_4$ was dissolved. At this time, the total alkali metal salt concentration was 510 mM in which PB was 10 mM and $K_2SO_4$ was 500 mM. The obtained liquid containing $K_2SO_4$ was used as the PB, and PEG was dissolved therein at a concentration of 100 g/L to prepare a first solution and a second solution. The first solution (total alkali metal salt concentration: 510 mM, PEG concentration: 100 g/L) and the second solution (total alkali metal salt concentration: 510 mM, PEG concentration: 100 g/L) thus prepared were mixed in the same volume ratio in an empty centrifuge tube. This mixed solution was used as a gelling solution containing PEG at a concentration of 100 g/L and an alkali metal salt at a concentration of 510 mM. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged. This result suggests that the formation of the porous structure does not depend on the type of the alkali metal salt.

<Example 6>

In Example 6, sodium dihydrogen phosphate contained in PB and potassium sulfate ($K_2SO_4$), which is an alkali metal salt different from disodium hydrogen phosphate, were additionally added to PB in advance. Specifically, $K_2SO_4$ was added as a solid to PB prepared so as to have an alkali metal salt concentration of 200 mM so that the $K_2SO_4$ concentration was 100 mM, and $K_2SO_4$ was dissolved. At this time, the total alkali metal salt concentration was 300 mM in which PB was 200 mM and $K_2SO_4$ was 100 mM. The obtained liquid containing $K_2SO_4$ was used as the PB, and PEG was dissolved therein at a concentration of 250 g/L to prepare a first solution and a second solution. The first solution (total alkali metal salt concentration: 300 mM, PEG concentration: 250 g/L) and the second solution (total alkali metal salt concentration: 300 mM, PEG concentration: 250 g/L) thus prepared were mixed in the same volume ratio in an empty centrifuge tube. This mixed solution was used as a gelling solution containing PEG at a concentration of 250 g/L and an alkali metal salt at a concentration of 300 mM. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged. This result indicates that a porous structure can be induced at a high PEG concentration (for example, 250 g/L) even when the total alkali metal salt concentration is 300 mM.

3. Preparation of Hydrogel Using Bi-Branched PEG and Tetra-Branched PEG

As raw material polymers, Linear-PEG-SH (linear sulfhydryl-polyethylene glycol; NOF CORPORATION, trade name DE-100SH) having a -SH group at a terminal and Tetra-PEG-OSu (tetrasuccinimidyl-polyethylene glycol; NOF CORPORATION, trade name PTE-100HS) having a succinimidyl group at a terminal were used. The weight average molecular weight (Mw) of both the raw material polymers was 10000.

Preparation of First Solution

Linear-PEG-SH was dissolved in the phosphate buffer solution (PB). At this time, the alkali metal salt concentration of the PB was set to 400 mM. The PEG concentration was set to 200 g/L.

Preparation of Second Solution

Tetra-PEG-OSu was dissolved in the PB. At this time, the alkali metal salt concentration of the PB was set to 400 mM. The PEG concentration was set to 100 g/L.

<Example 7>

The first solution (alkali metal salt concentration: 400 mM, PEG concentration: 200 g/L) and the second solution (alkali metal salt concentration: 400 mM, PEG concentration: 100 g/L) were mixed in the same volume ratio in an empty

centrifuge tube. This mixed solution was used as a gelling solution containing PEG at a concentration of 150 g/L and an alkali metal salt at a concentration of 400 mM. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged. This result indicates that the formation of the porous structure does not depend on the number of branches of the polymer constituting the hydrogel.

4. Preparation of Hydrogel Using Tetra-Branched PEG and Peptide

As one of raw material polymers, Tetra-PEG-OSu (tetrasuccinimidyl-polyethylene glycol-polyethylene glycol; NOF CORPORATION, trade name PTE-100HS) having a succinimidyl group at a terminal was used. The weight average molecular weight (Mw) was 10000. Trilysine (Lys-Lys-Lys, Sigma-Aldrich), which is a kind of peptide, was used as a substance reacting with Tetra-PEG-OSu. The molecular weight of trilysine is 402.53 g/mol.

Preparation of First Solution

Trilysine was dissolved in the phosphate buffer solution (PB). At this time, the alkali metal salt concentration of the PB was set to 400 mM. The trilysine concentration was set to 16.10 g/L.

Preparation of Second Solution

Tetra-PEG-OSu was dissolved in the PB. At this time, the alkali metal salt concentration of the PB was set to 400 mM. The PEG concentration was set to 100 g/L.

<Example 8>

The first solution (alkali metal salt concentration: 400 mM, trilysine concentration: 16.10 g/L) and the second solution (alkali metal salt concentration: 400 mM, PEG concentration: 100 g/L) were mixed in the same volume ratio in an empty centrifuge tube to obtain a gelling solution. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged. This result indicates that a porous structure is obtained if either the first solution or the second solution is composed of PEG.

5. Preparation of Hydrogel Using Tetra-Branched PEG and Protein

As one of raw material polymers, Tetra-PEG-OSu (tetrasuccinimidyl-polyethylene glycol-polyethylene glycol; NOF CORPORATION, trade name PTE-100HS) having a succinimidyl group at a terminal was used. The weight average molecular weight (Mw) was 10000. Gelatin (Nippi, Incorporated, trade name APAT), which is a kind of protein, was used as a substance reacting with Tetra-PEG-OSu. The molecular weight of gelatin is 60000 g/mol as a manufacturer's nominal value.

Preparation of First Solution

$K_2SO_4$ was added as a solid to a phosphate buffer solution (PB) prepared so as to have an alkali metal salt concentration of 200 mM so that the $K_2SO_4$ concentration was 500 mM, and $K_2SO_4$ was dissolved. At this time, the total alkali metal salt concentration was 700 mM in which PB was 200 mM and $K_2SO_4$ was 500 mM. Gelatin was dissolved in the liquid. The gelatin concentration was set to 24 g/L.

Preparation of Second Solution

$K_2SO_4$ was added as a solid to PB prepared so as to have an alkali metal salt concentration of 200 mM so that the

$K_2SO_4$ concentration was 500 mM, and $K_2SO_4$ was dissolved. At this time, the total alkali metal salt concentration was 700 mM in which PB was 200 mM and $K_2SO_4$ was 500 mM. Tetra-PEG-OSu was dissolved in the liquid. The PEG concentration was set to 100 g/L.

<Example 9>

The first solution (total alkali metal salt concentration: 700 mM, gelatin concentration: 24 g/L) and the second solution (total alkali metal salt concentration: 700 mM, PEG concentration: 100 g/L) were mixed in the same volume ratio in an empty centrifuge tube to obtain a gelling solution. The gelling solution (1 mL) was put into a 2.5 mL syringe barrel to which a 27G syringe needle (inner diameter: 0.22 ± 0.03) was connected, and discharged into a glass vial. It was visually confirmed that the gelling solution was liquid for at least 5 seconds from the completion of the preparation of the gelling solution to the completion of the discharge. Thereafter, the gelling solution contained in the glass vial was allowed to stand still for 24 hours without any special external stimulus. When the glass vial was tilted, the gelling solution lost fluidity and became a white solid. When the obtained hydrogel was compressed with a finger, it was observed that water was discharged. This result indicates that a porous structure can be imparted even when a protein is crosslinked with PEG to obtain a hydrogel.

6. Preparation of Hydrogel Using Tetra-Branched PEG and Polysaccharide

As one of raw material polymers, Tetra-PEG-OSu (tetrasuccinimidyl-polyethylene glycol-polyethylene glycol; XIAMEN SINOPEG BIOTECH CO., LTD., trade name 4-arm PEG-SC) having a succinimidyl group at a terminal was used. The weight average molecular weight (Mw) was 10000. A polysaccharide was used as a substance reacting with Tetra-PEG-OSu. Examples of the polysaccharide include chitosan and hyaluronic acid, but herein, as an example, chitosan (Santa Cruz Biotechnology, Inc., trade name Carboxymethyl chitosan) was used.

Preparation of First Solution

$K_2SO_4$ was added as a solid to a phosphate buffer solution (PB) prepared so as to have an alkali metal salt concentration of 200 mM so that the $K_2SO_4$ concentration was 0 to 500 mM, and $K_2SO_4$ was dissolved. At this time, the total alkali metal salt concentration was 200 to 700 mM in which PB was 200 mM and $K_2SO_4$ was 0 to 500 mM. Chitosan was dissolved in the liquid. The chitosan concentration was set to 60 g/L.

Preparation of Second Solution

$K_2SO_4$ was added as a solid to PB prepared so as to have an alkali metal salt concentration of 200 mM so that the $K_2SO_4$ concentration was 0 to 500 mM, and $K_2SO_4$ was dissolved. At this time, the total alkali metal salt concentration was 200 to 700 mM in which PB was 200 mM and $K_2SO_4$ was 0 to 500 mM. Tetra-PEG-OSu was dissolved in the liquid. The PEG concentration was set to 120 g/L.

<Comparative Example 3>

The first solution (total alkali metal salt concentration: 200 mM, chitosan concentration: 60 g/L) and the second solution (total alkali metal salt concentration: 200 mM, PEG concentration: 120 g/L) were mixed in the same volume ratio in an empty centrifuge tube to obtain a gelling solution. The gelling solution (1 mL) was put into a 2.5 mL syringe barrel to which a 27G syringe needle (inner diameter: 0.22 ± 0.03) was connected, and discharged into a Teflon container. It was visually confirmed that the gelling solution was liquid for at least 5 seconds from the completion of the preparation of the gelling solution to the completion of the discharge. Thereafter, the gelling solution contained in the Teflon container was allowed to stand still for 24 hours without any special external stimulus. When the Teflon container was tilted, the gelling solution lost fluidity and became a transparent solid. The obtained transparent hydrogel did not discharge water even when compressed with a finger.

<Example 10>

The first solution (total alkali metal salt concentration: 600 mM, chitosan concentration: 60 g/L) and the second solution (total alkali metal salt concentration: 600 mM, PEG concentration: 120 g/L) were mixed in the same volume ratio in an empty centrifuge tube to obtain a gelling solution. The gelling solution (1 mL) was put into a 2.5 mL syringe barrel to which a 27G syringe needle (inner diameter: 0.22 ± 0.03) was connected, and discharged into a Teflon container. It was visually confirmed that the gelling solution was liquid for at least 5 seconds from the completion of the preparation of the

gelling solution to the completion of the discharge. Thereafter, the gelling solution contained in the Teflon container was allowed to stand still for 24 hours without any special external stimulus. When the Teflon container was tilted, the gelling solution lost fluidity and became a white solid. When the obtained hydrogel was compressed with a finger, it was observed that water was discharged. This result indicates that a porous structure can be imparted even when a polysaccharide is crosslinked with PEG to obtain a hydrogel.

7. Measurement of Transmittance of Hydrogel

As raw material polymers, Tetra-PEG-SH (tetrasulfhydryl-polyethylene glycol; NOF CORPORATION, trade name PTE-100SH) having a -SH group at a terminal and Tetra-PEG-OSu (tetrasuccinimidyl-polyethylene glycol; NOF CORPORATION, trade name PTE-100HS) having a succinimidyl group at a terminal were used. The weight average molecular weight (Mw) of both the raw material polymers was 10000.

Preparation of First Solution

$K_2SO_4$ was added as a solid to a phosphate buffer solution (PB) prepared so as to have an alkali metal salt concentration of 200 mM so that the $K_2SO_4$ concentration was any concentration of 0 to 300 mM, and $K_2SO_4$ was dissolved. At this time, the total alkali metal salt concentration was 200 to 500 mM in which PB was 200 mM and $K_2SO_4$ was 0 to 300 mM. Tetra-PEG-SH was dissolved in the liquid. The PEG concentration was set to any concentration of 20 to 100 g/L.

Preparation of Second Solution

$K_2SO_4$ was added as a solid to PB prepared so as to have an alkali metal salt concentration of 200 mM so that the $K_2SO_4$ concentration was any concentration of 0 to 300 mM, and $K_2SO_4$ was dissolved. At this time, the total alkali metal salt concentration was 200 to 500 mM in which PB was 200 mM and $K_2SO_4$ was 0 to 300 mM. Tetra-PEG-OSu was dissolved in the liquid. The PEG concentration was set to any concentration of 20 to 100 g/L.

A pair of the first solution (total alkali metal salt concentration: 200 to 500 mM, PEG concentration: 20 to 100 g/L) and the second solution (total alkali metal salt concentration: 200 to 500 mM, PEG concentration: 20 to 100 g/L) having the same total alkali metal salt concentration and PEG concentration were mixed at the same volume ratio in an empty tube to obtain a gelling solution. 200 μL of each of the gelling solutions was poured into a 96-well microplate and left to stand still at 25°C for 24 hours. Thereafter, a photograph of the hydrogel formed in each well was taken (Fig. 3), and then the transmittance of each well was measured using Perkinelmer ARVO X3 (Fig. 4). The degree of cloudiness with the naked eye in Fig. 3 and the transmittance in Fig. 4 were well correlated. At a PEG concentration of 50 g/L or more, it was found that when the total alkali metal salt concentration was 400 mM or more, the transmittance was significantly less than 80%, and a cloudy porous structure was obtained. Even when the PEG concentration was 20 g/L, cloudiness was confirmed at a total alkali metal salt concentration of 450 mM or more. In the hydrogel having a transmittance of 80% or more, even when the hydrogel was compressed with a finger, water was not discharged. On the other hand, in all of the cloudy hydrogels, when the hydrogel was compressed with a finger, it was observed that water was discharged. These results mean that porosity can be realized by increasing the total alkali metal salt concentration even when the PEG concentration is low.

8. Microscopic Structure Observation and Swelling Degree Measurement of Hydrogel

As raw material polymers, Tetra-PEG-SH (tetrasulfhydryl-polyethylene glycol; NOF CORPORATION, trade name PTE-100SH) having a -SH group at a terminal and Tetra-PEG-OSu (tetrasuccinimidyl-polyethylene glycol; NOF CORPORATION, trade name PTE-100HS) having a succinimidyl group at a terminal were used. The weight average molecular weight (Mw) of both the raw material polymers was 10000.

Preparation of First Solution

$K_2SO_4$ was added as a solid to a phosphate buffer solution (PB) prepared so as to have an alkali metal salt concentration of 200 mM so that the $K_2SO_4$ concentration was any concentration of 0 to 300 mM, and $K_2SO_4$ was dissolved. At this time, the total alkali metal salt concentration was 200 to 500 mM in which PB was 200 mM and $K_2SO_4$ was 0 to 300 mM. Tetra-PEG-SH was dissolved in the liquid. The PEG concentration was set to 100 g/L. Alexa Fluor™ 594 $C_5$ Maleimide dissolved in dimethyl sulfoxide so as to be 1 mg/mL was additionally added to the prepared Tetra-PEG-SH solution at 1 vol% of the Tetra-PEG-SH solution.

Preparation of Second Solution

$K_2SO_4$ was added as a solid to PB prepared so as to have an alkali metal salt concentration of 200 mM so that the $K_2SO_4$ concentration was any concentration of 0 to 300 mM, and $K_2SO_4$ was dissolved. At this time, the total alkali metal salt concentration was 200 to 500 mM in which PB was 200 mM and $K_2SO_4$ was 0 to 300 mM. Tetra-PEG-OSu was dissolved in the liquid. The PEG concentration was set to 100 g/L.

A pair of the first solution (total alkali metal salt concentration: 200 to 500 mM, PEG concentration: 100 g/L) and the second solution (total alkali metal salt concentration: 200 to 500 mM, PEG concentration: 100 g/L) having the same total alkali metal salt concentration and PEG concentration were mixed at the same volume ratio in an empty tube to obtain a gelling solution. Each gelling solution was poured into a cylindrical mold made of polytetrafluoroethylene (diameter: 15 mm, height: 7 mm) and left to stand still at 25°C for 24 hours. Thereafter, the hydrogel was removed from the mold. This state was defined as "as-prepared state", and a photograph of each hydrogel was taken (the upper part of Fig. 5). Next, a confocal microscope image of each hydrogel was taken at an excitation wavelength of 590 nm and a fluorescence wavelength of 617 nm with a confocal microscope LSM 800 (ZEISS) (the lower part of Fig. 5). Subsequently, the hydrogel was immersed in phosphate buffered saline (FUJIFILM Wako Pure Chemical Corporation) for 24 hours. This state was defined as "equilibrium state", and a photograph of each hydrogel was taken again (the upper part of Fig. 6). Similarly, a confocal microscope image of each hydrogel was also taken (the lower part of Fig. 6). The cloudy state and the porous structure were maintained even in the equilibrium state. It was found that at a high total alkali metal salt concentration, a porous structure was formed in the order of $\mu$m, and the structure was stored for a long time even in water.

The equilibrium swelling degree Q of the hydrogel was defined as $(d_eq/d_0)^3$ and plotted (Fig. 7). "d_eq" is the diameter of the hydrogel in an equilibrium state, and "d_0" is the diameter of the hydrogel immediately after preparation. It was recognized that at a total alkali metal salt concentration of up to 350 mM, the equilibrium swelling degree was relatively flat, whereas at a total alkali metal salt concentration of 400 mM or more, the equilibrium swelling degree tended to increase. Specifically, when the value at a total alkali metal salt concentration of 200 mM was designated as Q_low, the value was 1.1*Q_low or more at a total alkali metal salt concentration of 400 mM or more. This corresponds well to the result of the previous microscopic structure observation, and thus it is considered that the generated porous structure affected the value. That is, the presence of the microscopic porous structure can be indirectly determined from the equilibrium swelling degree.

9. Measurement of Tensile Stress at Break of Hydrogel

As raw material polymers, Tetra-PEG-SH (tetrasulfhydryl-polyethylene glycol; NOF CORPORATION, trade name PTE-100SH) having a -SH group at a terminal and Tetra-PEG-OSu (tetrasuccinimidyl-polyethylene glycol; NOF CORPORATION, trade name PTE-100HS) having a succinimidyl group at a terminal were used. The weight average molecular weight (Mw) of both the raw material polymers was 10000.

Preparation of First Solution

$K_2SO_4$ was added as a solid to a phosphate buffer solution (PB) prepared so as to have an alkali metal salt concentration of 200 mM so that the $K_2SO_4$ concentration was any concentration of 0 to 250 mM, and $K_2SO_4$ was dissolved. At this time, the total alkali metal salt concentration was 200 to 450 mM in which PB was 200 mM and $K_2SO_4$ was 0 to 250 mM. Tetra-PEG-SH was dissolved in the liquid. The PEG concentration was set to 100 g/L.

Preparation of Second Solution

$K_2SO_4$ was added as a solid to PB prepared so as to have an alkali metal salt concentration of 200 mM so that the $K_2SO_4$ concentration was any concentration of 0 to 250 mM, and $K_2SO_4$ was dissolved. At this time, the total alkali metal salt concentration was 200 to 450 mM in which PB was 200 mM and $K_2SO_4$ was 0 to 250 mM. Tetra-PEG-OSu was dissolved in the liquid. The PEG concentration was set to 100 g/L.

A pair of the first solution (total alkali metal salt concentration: 200 to 450 mM, PEG concentration: 100 g/L) and the second solution (total alkali metal salt concentration: 200 to 450 mM, PEG concentration: 100 g/L) having the same total alkali metal salt concentration and PEG concentration were mixed at the same volume ratio in an empty tube to obtain a gelling solution. Each gelling solution was poured into a dumbbell-shaped silicone mold defined in JIS K 6251 and left to stand still at 25°C for 24 hours. Thereafter, the hydrogel was removed from the mold. Each hydrogel was subjected to a tensile test with Autograph AG-X plus (SHIMADZU CORPORATION), and the stress $\sigma$ at break (tensile stress at break) was recorded. The same test was performed five times for each hydrogel, and the average value and the standard deviation thereof were plotted (Fig. 8). It was recognized that at a total alkali metal salt concentration of up to 350 mM, the tensile stress at break was relatively flat, whereas at a total alkali metal salt concentration of 400 mM or more, the tensile

stress at break tended to increase. Specifically, when the value at a total alkali metal salt concentration of 200 mM was designated as σ_low, the value was 1.5*σ_low or more at a total alkali metal salt concentration of 400 mM or more. This corresponds well to the result of the previous microscopic structure observation, and thus it is considered that the generated porous structure affected the value. That is, the presence of the microscopic porous structure can be indirectly determined from the tensile stress at break.

## Claims

**1.** A method for producing a hydrogel having a porous structure, the production method comprising steps of:

a) preparing a first solution in which a compound A is dissolved in an aqueous solvent containing an alkali metal salt at a concentration of 0 to 1000 mM;
b) preparing a second solution in which a compound B chemically crosslinkable with the compound A is dissolved in an aqueous solvent containing an alkali metal salt at a concentration of 0 to 1000 mM;
c) mixing the first solution and the second solution to obtain a gelling solution containing one or more alkali metal salts at a concentration of 300 mM to 1000 mM; and
d) forming a chemical crosslinking by the compound A and the compound B in the gelling solution regardless of the presence or absence of an external stimulus to obtain a hydrogel having a porous structure,

wherein at least one of the compounds A and B is a linear, tri-branched, tetra-branched, or octa-branched polyethylene glycol (PEG) having a total of two or more nucleophilic functional groups or electrophilic functional groups in a side chain or at a terminal,
wherein the weight average molecular weight of the PEG is in the range of $2\times10^3$ to $100\times10^3$ Da, and
wherein a time from preparation of the gelling solution to solidification of the gelling solution is 1 second or longer.

**2.** The production method according to claim 1, wherein the alkali metal salt is selected from the group consisting of sodium dihydrogen phosphate, disodium hydrogen phosphate, sodium citrate, sodium acetate, potassium sulfate, sodium sulfate, and any combination thereof.

**3.** The production method according to claim 1 or claim 2, wherein the hydrogel has a maximum value of transmittance at a wavelength of 405 nm of 80% or less.

**4.** The production method according to any one of claims 1 to 3, wherein the hydrogel contains the polyethylene glycol in a range of 20 g/L to 250 g/L.

**5.** The production method according to any one of claims 1 to 4, wherein when in a mixed solution of the first solution and the second solution, an equilibrium swelling degree of a hydrogel formed under a condition of a concentration of the alkali metal salt of 200 mM is designated as $Q_{low}$, and an equilibrium swelling degree of a hydrogel formed in the presence of an arbitrary concentration within 300 mM to 1000 mM of the alkali metal salt is designated as Q, Q satisfies the following relational expression:

$$Q \geq 1.1*Q_{low},$$

wherein the equilibrium swelling degree is defined as $(d_eq/d_0)^3$,
wherein "d_eq" is the diameter of the hydrogel in an equilibrium state, and "d_0" is the diameter of the hydrogel immediately after preparation.

**6.** The production method according to any one of claims 1 to 5, wherein when in a mixed solution of the first solution and the second solution, a tensile stress at break of a hydrogel formed under a condition of a concentration of the alkali metal salt of 200 mM is designated as $\sigma_{low}$, and a tensile stress at break of a hydrogel formed in the presence of an arbitrary concentration within 300 mM to 1000 mM of the alkali metal salt is designated as σ, σ satisfies the following relational expression:

$$\sigma \geq 1.5*\sigma_{low},$$

wherein the tensile stress at break is measured by means of a tensile test.

7. The production method according to any one of claims 1 to 6, wherein one of the compounds A and B is PEG and the other is composed of one or more selected from the group consisting of PEG, a peptide, a protein, chitosan, hyaluronic acid, and derivatives thereof.

8. The production method according to any one of claims 1 to 7, wherein the external stimulus in the step d) is light irradiation.

9. A kit for producing a hydrogel having a porous structure, the kit comprising:

a first solution in which a compound A is dissolved in an aqueous solvent containing an alkali metal salt at a concentration of 0 to 1000 mM; and
a second solution in which a compound B chemically crosslinkable with the compound A is dissolved in an aqueous solvent containing an alkali metal salt at a concentration of 0 to 1000 mM,
wherein a concentration of the alkali metal salt when the first solution and the second solution are mixed is in a range of 300 mM to 1000 mM in total, and
at least one of the compounds A and B is a linear, tri-branched, tetra-branched, or octa-branched polyethylene glycol (PEG) having a total of two or more nucleophilic functional groups or electrophilic functional groups in a side chain or at a terminal,
wherein the weight average molecular weight of the PEG is in the range of $2\times10^3$ to $100x10^3$ Da, and
wherein a time from preparation of a mixture of the first and second solutions to solidification of the mixture of the first and second solutions is 1 second or longer.

## Patentansprüche

1. Verfahren zur Herstellung eines Hydrogels mit einer porösen Struktur, wobei das Herstellungsverfahren die folgenden Schritte umfasst:

a) Ansetzen einer ersten Lösung, in der eine Verbindung A in einem wässrigen Lösungsmittel gelöst wird, das ein Alkalimetallsalz in einer Konzentration von 0 bis 1000 mM enthält;
b) Ansetzen einer zweiten Lösung, in der eine mit der Verbindung A chemisch vernetzbare Verbindung B in einem wässrigen Lösungsmittel gelöst wird, das ein Alkalimetallsalz in einer Konzentration von 0 bis 1000 mM enthält;
c) Mischen der ersten Lösung und der zweiten Lösung, um eine Gelierlösung zu erhalten, die ein oder mehrere Alkalimetallsalze in einer Konzentration von 300 mM bis 1000 mM enthält; und
d) Bilden einer chemischen Vernetzung durch die Verbindung A und die Verbindung B in der Gelierlösung unabhängig von dem Vorhandensein oder Fehlen eines externen Stimulus, um ein Hydrogel mit einer porösen Struktur zu erhalten,

wobei mindestens eine der Verbindungen A und B ein lineares, dreifach, vierfach oder achtfach verzweigtes Polyethylenglykol (PEG) mit insgesamt zwei oder mehr nukleophilen funktionellen Gruppen oder elektrophilen funktionellen Gruppen in einer Seitenkette oder an einem Endpunkt ist,
wobei das gewichtsmittlere Molekulargewicht des PEG im Bereich von $2\times10^3$ bis $100\times10^3$ Da liegt, und
wobei eine Zeit von dem Ansetzen der Gelierlösung bis zur Verfestigung der Gelierlösung 1 Sekunde oder länger ist.

2. Herstellungsverfahren nach Anspruch 1, wobei das Alkalimetallsalz ausgewählt ist aus der Gruppe bestehend aus Natriumdihydrogenphosphat, Dinatriumhydrogenphosphat, Natriumcitrat, Natriumacetat, Kaliumsulfat, Natriumsulfat und einer beliebigen Kombination davon.

3. Herstellungsverfahren nach Anspruch 1 oder Anspruch 2, wobei das Hydrogel einen maximalen Wert der Durchlässigkeit bei einer Wellenlänge von 405 nm von 80 % oder weniger aufweist.

4. Herstellungsverfahren nach einem der Ansprüche 1 bis 3, wobei das Hydrogel das Polyethylenglykol in einem Bereich von 20 g/l bis 250 g/l enthält.

5. Herstellungsverfahren nach einem der Ansprüche 1 bis 4, wobei in einer gemischten Lösung der ersten Lösung und

der zweiten Lösung ein Gleichgewichtsquellgrad eines unter einer Bedingung einer Konzentration des Alkalimetallsalzes von 200 mM gebildeten Hydrogels als $Q_{low}$ bezeichnet wird und ein Gleichgewichtsquellgrad eines in Gegenwart einer beliebigen Konzentration innerhalb von 300 mM bis 1000 mM des Alkalimetallsalzes gebildeten Hydrogels als Q bezeichnet wird, wobei Q den folgenden relationalen Ausdruck erfüllt:

$$Q \geq 1{,}1 * Q_{low},$$

wobei der Gleichgewichtsschwellungsgrad definiert ist als

$$(d_eq/d_0)^3,$$

wobei "d_eq" der Durchmesser des Hydrogels in einem Gleichgewichtszustand ist und "d_0" der Durchmesser des Hydrogels unmittelbar nach dem Ansetzen ist.

6. Herstellungsverfahren nach einem der Ansprüche 1 bis 5, wobei in einer gemischten Lösung der ersten Lösung und der zweiten Lösung eine Bruchzugspannung eines unter einer Bedingung einer Konzentration des Alkalimetallsalzes von 200 mM gebildeten Hydrogels als $\sigma_{low}$ bezeichnet wird und eine Bruchzugspannung eines in Gegenwart einer beliebigen Konzentration innerhalb von 300 mM bis 1000 mM des Alkalimetallsalzes gebildeten Hydrogels als $\sigma$ bezeichnet wird, wobei $\sigma$ den folgenden relationalen Ausdruck erfüllt:

$$\sigma \geq 1{,}5 * \sigma_{low},$$

wobei die Bruchzugspannung mittels eines Zugversuchs gemessen wird.

7. Herstellungsverfahren nach einem der Ansprüche 1 bis 6, wobei eine der Verbindungen A und B PEG ist und die andere aus einem oder mehreren ausgewählt aus der Gruppe bestehend aus PEG, einem Peptid, einem Protein, Chitosan, Hyaluronsäure und Derivaten davon zusammengesetzt ist.

8. Herstellungsverfahren nach einem der Ansprüche 1 bis 7, wobei der externe Stimulus in Schritt d) eine Lichteinstrahlung ist.

9. Kit zur Herstellung eines Hydrogels mit einer porösen Struktur, wobei das Kit Folgendes umfasst:

eine erste Lösung, in der eine Verbindung A in einem wässrigen Lösungsmittel gelöst wird, das ein Alkalimetallsalz in einer Konzentration von 0 bis 1000 mM enthält; und
eine zweite Lösung, in der eine mit der Verbindung A chemisch vernetzbare Verbindung B in einem wässrigen Lösungsmittel gelöst wird, das ein Alkalimetallsalz in einer Konzentration von 0 bis 1000 mM enthält,
wobei eine Konzentration des Alkalimetallsalzes, wenn die erste Lösung und die zweite Lösung gemischt werden, insgesamt in einem Bereich von 300 mM bis 1000 mM liegt, und
mindestens eine der Verbindungen A und B ein lineares, dreifach, vierfach oder achtfach verzweigtes Polyethylenglykol (PEG) mit insgesamt zwei oder mehr nukleophilen funktionellen Gruppen oder elektrophilen funktionellen Gruppen in einer Seitenkette oder an einem Endpunkt ist,
wobei das gewichtsmittlere Molekulargewicht des PEG im Bereich von $2 \times 10^3$ bis $100 \times 10^3$ Da liegt, und
wobei eine Zeit von dem Ansetzen einer Mischung aus der ersten und zweiten Lösung bis zur Verfestigung der Mischung aus der ersten und zweiten Lösung 1 Sekunde oder länger ist.

## Revendications

1. Procédé de production d'un hydrogel à structure poreuse, le procédé de production comprenant les étapes de :

a) préparation d'une première solution dans laquelle un composé A est dissous dans un solvant aqueux contenant un sel de métal alcalin à une concentration de 0 à 1000 mM ;
b) préparation d'une seconde solution dans laquelle un composé B chimiquement réticulable avec le composé A est dissous dans un solvant aqueux contenant un sel de métal alcalin à une concentration de 0 à 1000 mM ;
c) mélange de la première solution et la seconde solution pour obtenir une solution gélifiante contenant un ou

plusieurs sels de métaux alcalins à une concentration de 300 mM à 1000 mM ; et
d) formation d'une réticulation chimique par le composé A et le composé B dans la solution gélifiante indépendamment de la présence ou l'absence d'un stimulus externe pour obtenir un hydrogel à structure poreuse,

dans lequel au moins l'un des composés A et B est un polyéthylène glycol (PEG) linéaire, tri-ramifié, tétra-ramifié ou octa-ramifié comportant un total de deux ou plus groupes fonctionnels nucléophiles ou groupes fonctionnels électrophiles dans une chaîne latérale ou en position terminale,
dans lequel le poids moléculaire moyen en poids du PEG est compris dans la plage de $2 \times 10^3$ à $100 \times 10^3$ Da, et
dans lequel le temps entre la préparation de la solution gélifiante et la solidification de la solution gélifiante est de 1 seconde ou plus.

**2.** Procédé de production selon la revendication 1, dans lequel le sel de métal alcalin est choisi dans le groupe constitué de dihydrogénophosphate de sodium, d'hydrogénophosphate disodique, de citrate de sodium, d'acétate de sodium, de sulfate de potassium, de sulfate de sodium et de toute combinaison de ceux-ci.

**3.** Procédé de production selon la revendication 1 ou la revendication 2, dans lequel l'hydrogel a une valeur maximale de transmittance à une longueur d'onde de 405 nm de 80 % ou moins.

**4.** Procédé de production selon l'une quelconque des revendications 1 à 3, dans lequel l'hydrogel contient le polyéthylène glycol dans une plage allant de 20 g/L à 250 g/L.

**5.** Procédé de production selon l'une quelconque des revendications 1 à 4, dans lequel, lorsqu'il est dans une solution mélangée de la première solution et de la seconde solution, un degré de gonflement d'équilibre d'un hydrogel formé dans une condition de concentration du sel de métal alcalin de 200 mM est désigné comme $Q_{low}$, et un degré de gonflement d'équilibre d'un hydrogel formé en présence d'une concentration arbitraire dans les 300 mM à 1000 mM du sel de métal alcalin est désigné comme Q, Q satisfait l'expression relationnelle suivante :

$$Q \geq 1.1 * Q_{low},$$

dans lequel le degré de gonflement d'équilibre est défini comme

$$(d_eq/d_0)^3,$$

dans lequel « d_eq » est le diamètre de l'hydrogel dans un état d'équilibre, et « d_0 » est le diamètre de l'hydrogel immédiatement après la préparation.

**6.** Procédé de production selon l'une quelconque des revendications 1 à 5, dans lequel, lorsqu'il est dans une solution mélangée de la première solution et de la seconde solution, une contrainte de traction à la rupture d'un hydrogel formé dans une condition de concentration du sel de métal alcalin de 200 mM est désignée comme $\sigma_{low}$, et une contrainte de traction à la rupture d'un hydrogel formé en présence d'une concentration arbitraire dans les 300 mM à 1000 mM du sel de métal alcalin est désignée comme $\sigma$, $\sigma$ satisfait l'expression relationnelle suivante :

$$\sigma \geq 1.5 * \sigma_{low},$$

dans lequel la contrainte de traction à la rupture est mesurée au moyen d'un test de traction.

**7.** Procédé de production selon l'une quelconque des revendications 1 à 6, dans lequel l'un des composés A et B est le PEG et l'autre est composé d'un ou de plusieurs choisis dans le groupe constitué par le PEG, un peptide, une protéine, le chitosane, l'acide hyaluronique et leurs dérivés.

**8.** Procédé de production selon l'une quelconque des revendications 1 à 7, dans lequel le stimulus externe à 'étape d) est une irradiation lumineuse.

**9.** Kit de production d'un hydrogel à structure poreuse, le kit comprenant :

une première solution dans laquelle un composé A est dissous dans un solvant aqueux contenant un sel de métal alcalin à une concentration de 0 à 1000 mM ; et

une seconde solution dans laquelle un composé B chimiquement réticulable avec le composé A est dissous dans un solvant aqueux contenant un sel de métal alcalin à une concentration de 0 à 1000 mM,

dans lequel une concentration du sel de métal alcalin lorsque la première solution et la seconde solution sont mélangées est comprise dans une plage de 300 mM à 1000 mM au total, et

au moins l'un des composés A et B est un polyéthylène glycol (PEG) linéaire, tri-ramifié, tétra-ramifié ou octa-ramifié comportant un total de deux ou plus groupes fonctionnels nucléophiles ou groupes fonctionnels électrophiles dans une chaîne latérale ou en position terminale,

dans lequel le poids moléculaire moyen en poids du PEG est compris dans la plage de $2\times10^3$ à $100\times10^3$ Da, et dans lequel le temps entre la préparation d'un mélange des première et seconde solutions et la solidification du mélange des première et seconde solutions est de 1 seconde ou plus.

FIG. 1

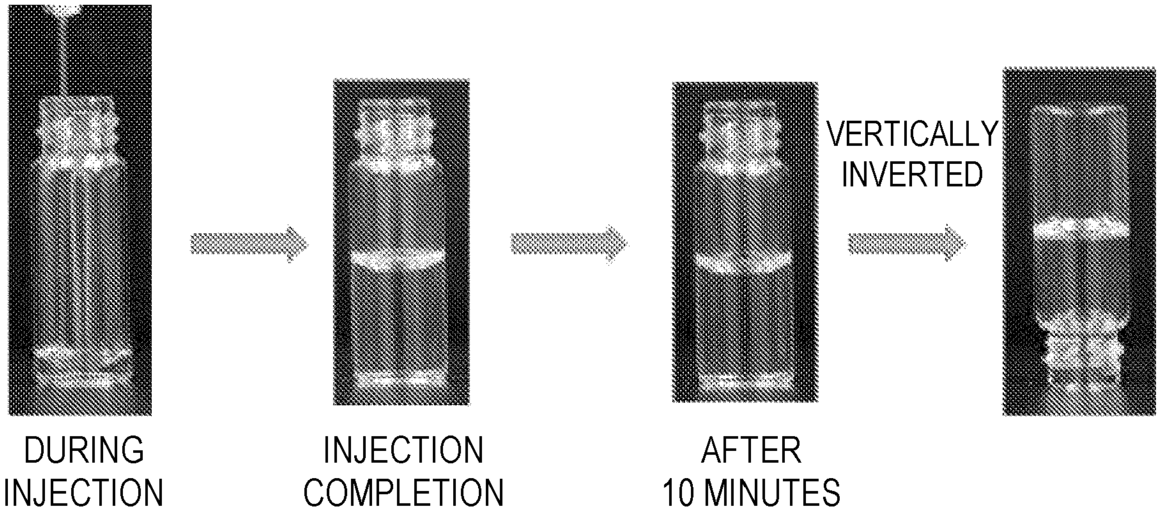
DURING INJECTION
INJECTION COMPLETION
AFTER 10 MINUTES
VERTICALLY INVERTED

FIG. 2

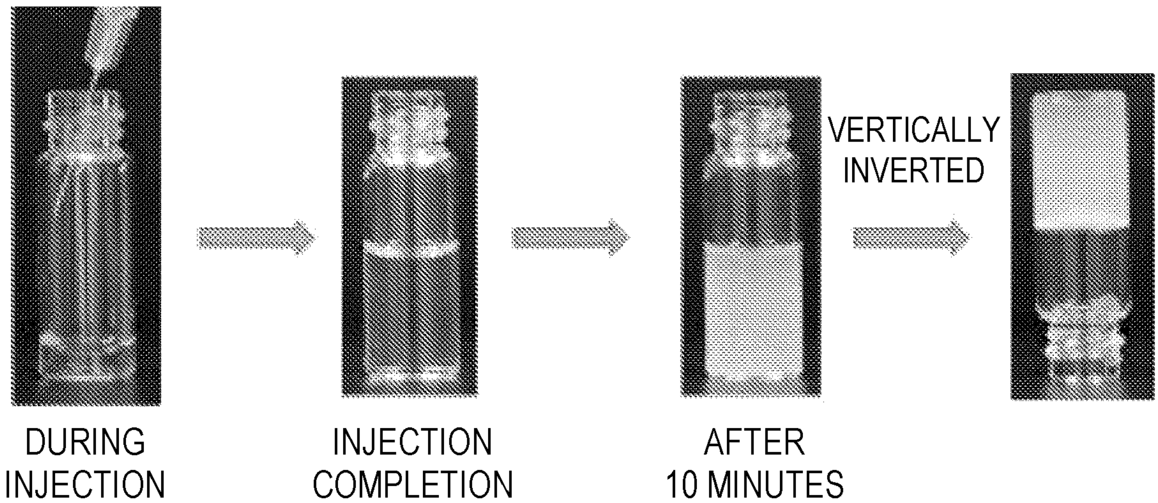
DURING INJECTION
INJECTION COMPLETION
AFTER 10 MINUTES
VERTICALLY INVERTED

FIG. 3

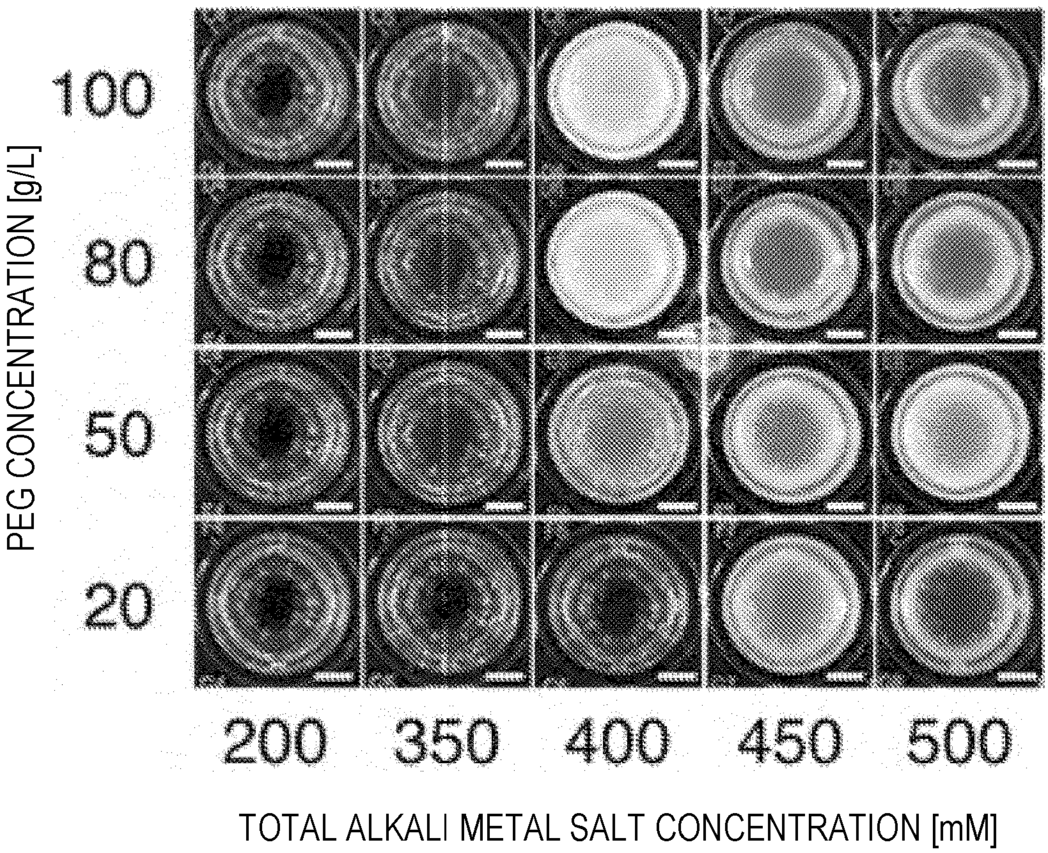
PEG CONCENTRATION [g/L]
100
80
50
20
200
350
400
450
500
TOTAL ALKALI METAL SALT CONCENTRATION [mM]

FIG. 4

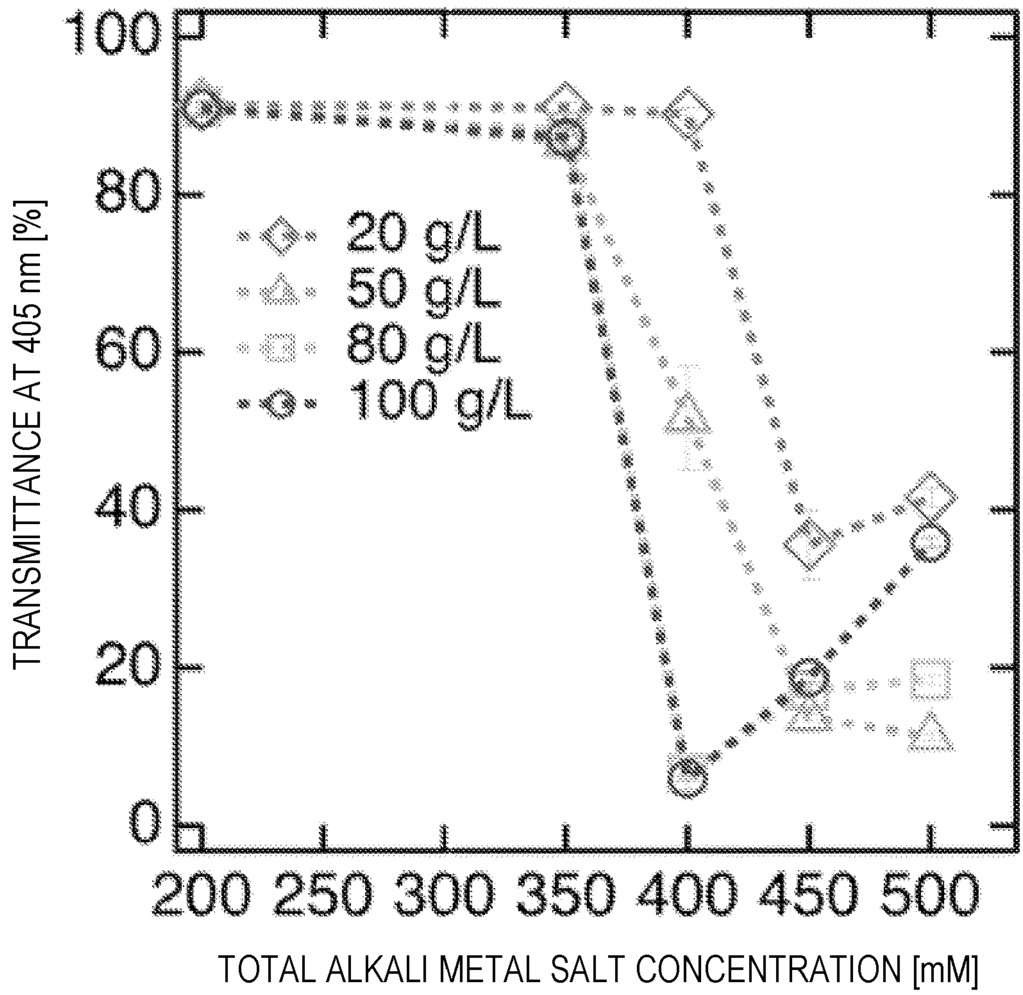
100
80
60
40
20
0
TRANSMITTANCE AT 405 nm [%]
20 g/L
50 g/L
80 g/L
100 g/L
200 250 300 350 400 450 500
TOTAL ALKALI METAL SALT CONCENTRATION [mM]

# FIG. 5

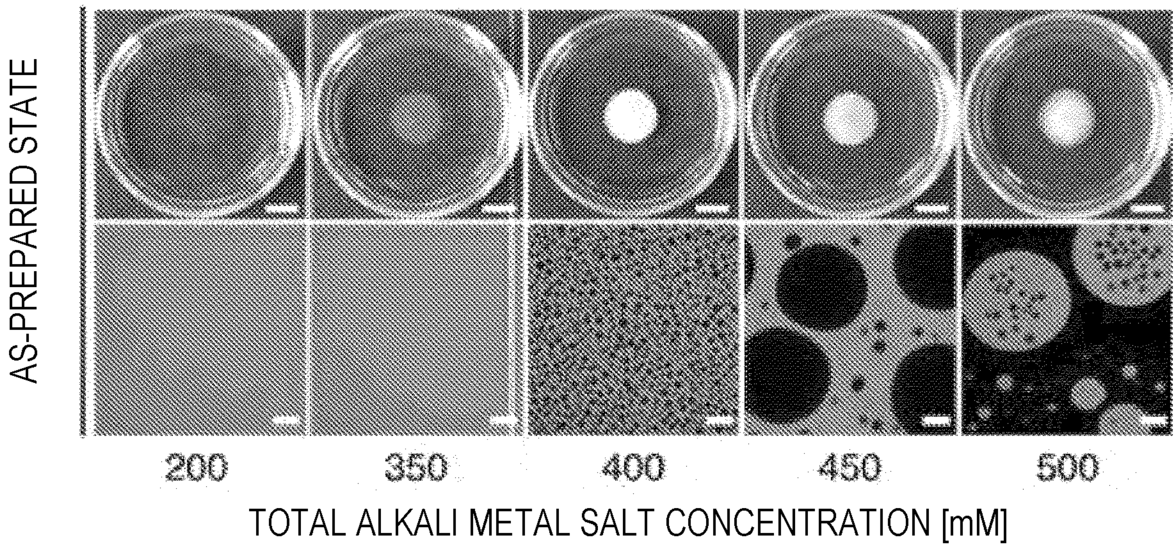
AS-PREPARED STATE
200
350
400
450
500
TOTAL ALKALI METAL SALT CONCENTRATION [mM]

# FIG. 6

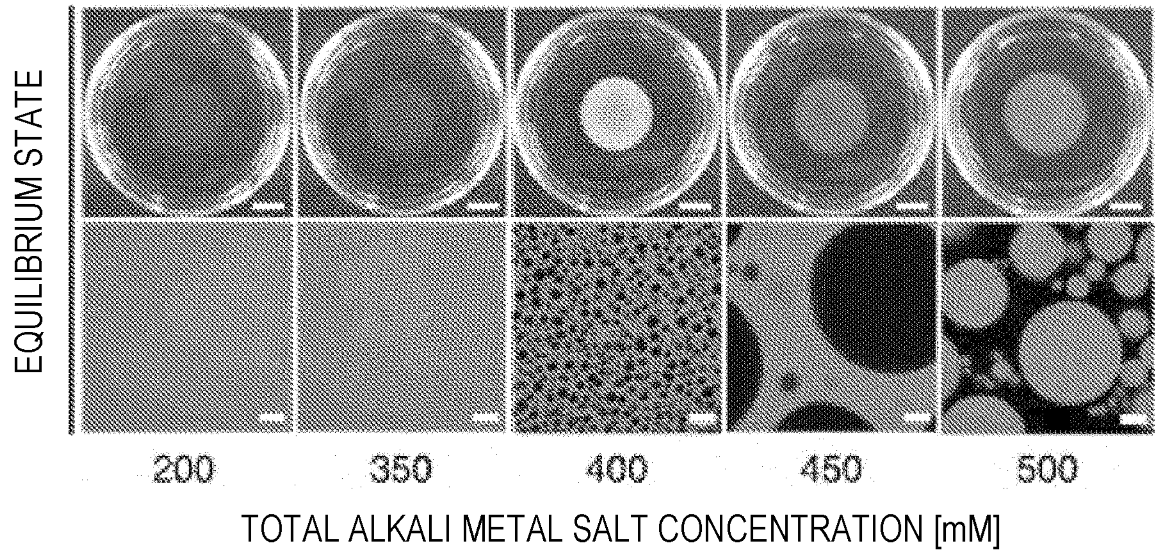
EQUILIBRIUM STATE
200
350
400
450
500
TOTAL ALKALI METAL SALT CONCENTRATION [mM]

FIG. 7

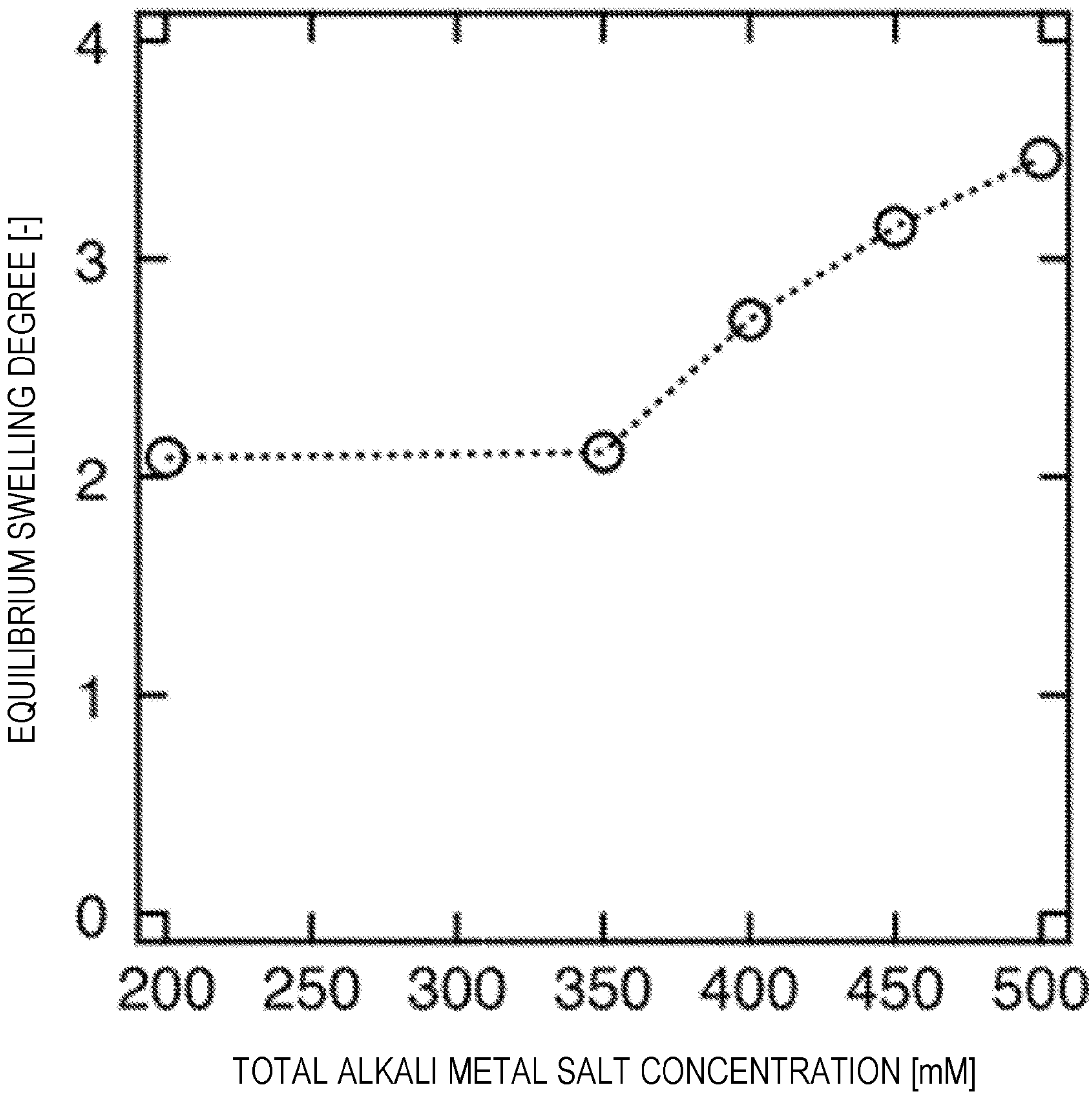
EQUILIBRIUM SWELLING DEGREE [-]
0
1
2
3
4
200
250
300
350
400
450
500
TOTAL ALKALI METAL SALT CONCENTRATION [mM]

FIG. 8

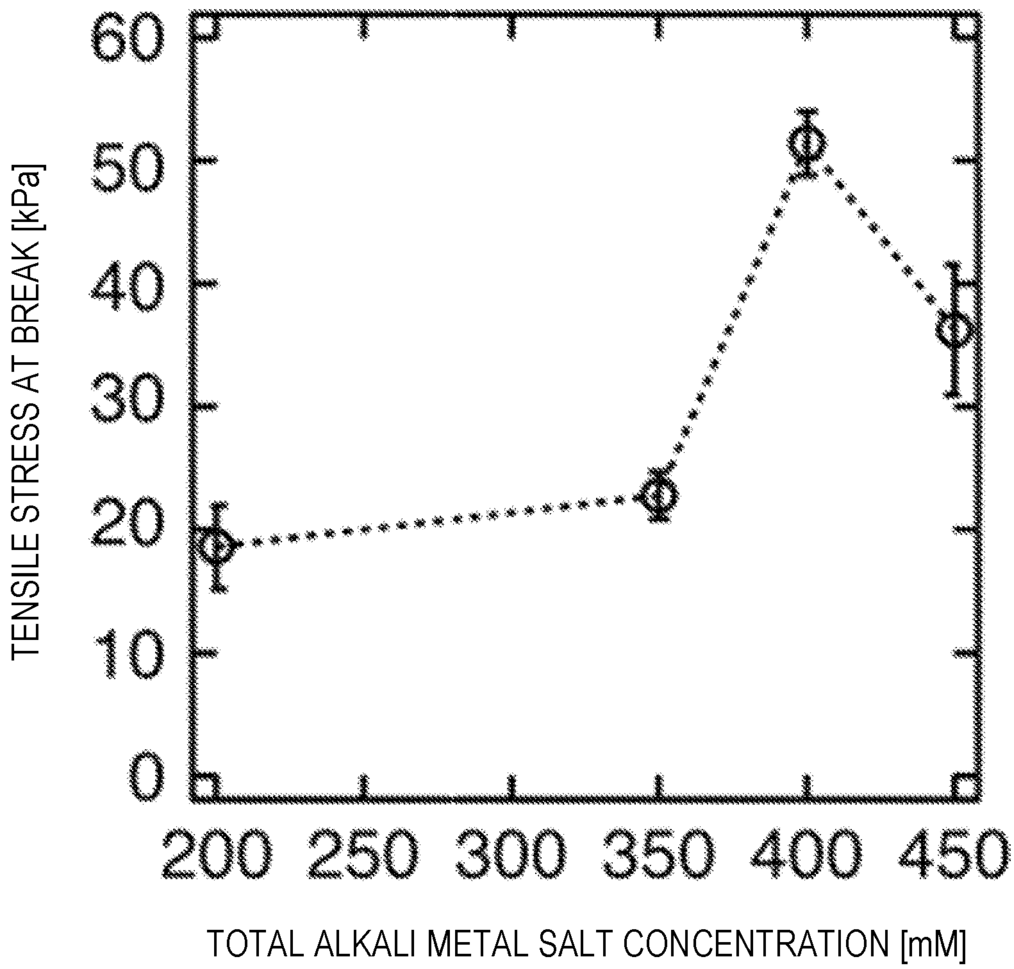
TENSILE STRESS AT BREAK [kPa]
0
10
20
30
40
50
60
200
250
300
350
400
450
TOTAL ALKALI METAL SALT CONCENTRATION [mM]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAKAI et al.** *Macromolecules*, 2008, vol. 41, 5379-5384
- **KATASHIMA et al.** *Polymer Journal*, 2019, vol. 51 (9), 851-859
- **MATSUNAGA et al.** *Macromolecules*, 2009, vol. 42 (4), 1344-1351
- **KURAKAZU et al.** *Macromolecules*, 2010, vol. 43 (8), 3935-3940
- **SAKAI et al.** *Macromolecules*, 2008, vol. 41 (14), 5379-5384